# EUROPÄISCHE PATENTSCHRIFT

(11) **EP 0 688 318 B1**
(45) Veröffentlichungstag und Bekanntmachung des Hinweises auf die Patenterteilung: **17.02.1999**
(21) Anmeldenummer: 93906496.0
(22) Anmeldetag: 10.03.1993
(51) Int. Cl.: C07D 213/84, C07D 213/85, C07D 405/12

(54) **VERFAHREN ZUR HERSTELLUNG VON DIAMINOPYRIDINEN**
PROCESS FOR PRODUCING DIAMINO PYRIDINES
PROCEDE DE FABRICATION DE DIAMINOPYRIDINES

(43) Veröffentlichungstag der Anmeldung: 27.12.1995
(73) Patentinhaber: BASF Aktiengesellschaft, 67063 Ludwigshafen (DE)
(72) Erfinder: LAMM, Gunther, D-6733 Hassloch (DE); LOEFFLER, Hermann, D-6720 Speyer (DE); REICHELT, Helmut, D-6730 Neustadt (DE)
(86) Internationale Anmeldenummer: EP9300538
(87) Internationale Veröffentlichungsnummer: WO9420469

(56) Entgegenhaltungen:
- DE-A- 2 916 319
- DE-A- 3 731 626
- DE-B- 2 260 827
- US-A- 3 980 659

## Beschreibung

Die vorliegende Erfindung betrifft ein neues Verfahren zur Herstellung von 2,6-Diaminocyanopyridinen durch Umsetzung der 2,6-Dichlorverbindungen mit Aminen.

Aus der US-A-3 853 895 ist die Herstellung von 2,6-Diaminopyridinen bekannt. Die dort beschriebene Herstellweise ist allerdings unbefriedigend, da zur Umsetzung große Mengen an Amin benötigt werden und außerdem die Zielprodukte nur in ungenügender Ausbeute anfallen.

Weiterhin beschreiben die DE-A-2 260 827, DE-A-2 916 319 und US-A-3 980 659 die Herstellung von 2,6-Diaminocyanopyridinen, wobei die entsprechenden Dichlor- oder Aminomonochlorverbindungen als Ausgangsprodukte dienen.

Aufgabe der vorliegenden Erfindung war es, ein neues Verfahren zur Herstellung von 2,6-Diaminocyanopyridinen bereitzustellen, das ebenfalls von den 2,6-Dichlorverbindungen ausgeht und die Zielprodukte in einfacher Weise und in hoher Ausbeute und Reinheit liefert.

Es wurde nun gefunden, daß die Herstellung von Diaminopyridinen der Formel I 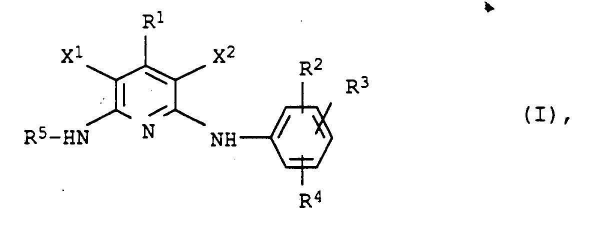 in der
einer der beiden Reste X¹ und X² Wasserstoff, C₁-C₄-Alkyl, Halogen oder Nitro und der andere Cyano,
- R¹: Wasserstoff, C₁-C₄-Alkyl oder Phenyl,
- R² und R³: unabhängig voneinander jeweils Wasserstoff, C₁-C₄-Alkyl oder C₁-C₄-Alkoxy,
- R⁴: Wasserstoff oder C₁-C₄-Alkyl und
- R⁵: C₁-C₁₀-Alkyl, das durch 1 bis 3 Sauerstoffatome in Etherfunktion unterbrochen sein kann und gegebenenfalls durch Hydroxy, C₁-C₄-Alkanoyloxy, Phenoxy, Phenyl, Tetrahydrofuranyl oder Tetrahydropyranyl substituiert ist, C₃-C₄-Alkenyl oder C₅-C₇-Cycloalkyl bedeuten,
durch Umsetzung von Dichlorpyridinen der Formel II 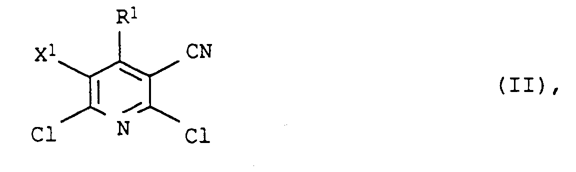 in der R¹ und X¹ jeweils die obengenannte Bedeutung besitzen, mit Aminen, wobei man in einem 1. Schritt ein Dichlorpyridin der Formel II mit einem Amin der Formel III

R⁵-NH₂ (III),

in der R⁵ die obengenannte Bedeutung besitzt, bei einer Temperatur von 10 bis 80°C in Gegenwart einer Base und eines inerten organischen Verdünnungsmittels und/oder Wasser umsetzt, anschließend vom Verdünnungsmittel befreit und danach, gegebenenfalls nach Zwischenisolierung des Reaktionsprodukts, in einem 2. Schritt mit einem Anilin der Formel IV 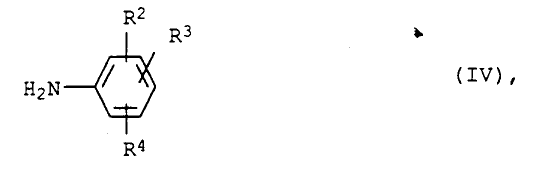 in der R², R³ und R⁴ jeweils die obengenannte Bedeutung besitzen, zur Reaktion bringt, vorteilhaft gelingt, wenn man den 2. Schritt in einer Schmelze bei einer Temperatur von 90 bis 165°C und bei einem pH-Wert von 3,5 bis 6,5 vornimmt, wobei je mol Dichlorpyridin II 1,3 bis 3 mol Anilin IV zur Anwendung kommen.

Alle in den obengenannten Formeln auftretenden Alkylgruppen können sowohl geradkettig als auch verzweigt sein.

Reste R¹, R², R³, R⁴, R⁵, X¹ und X² sind z.B. Methyl, Ethyl, Propyl, Isopropyl, Butyl, Isobutyl oder sec-Butyl.

Reste R⁴ sind weiterhin z.B. Methoxy, Ethoxy, Propoxy, Isopropoxy, Butoxy, Isobutoxy oder sec-Butoxy.

Reste R⁵ sind weiterhin z.B. Pentyl, Isopentyl, Neopentyl, tert-Pentyl, Hexyl, 2-Methylpentyl, Heptyl, Octyl, 2-Ethylhexyl, Isooctyl, Nonyl, Isononyl, Decyl, Benzyl, 1- oder 2-Phenylethyl, 2- oder 3-Phenylpropyl, 3-Phenylprop-2-yl, 2-Hydroxyethyl, 2-Hydroxypropyl, 3-Hydroxypropyl, 3-Hydroxyprop-2-yl, 2-Hydroxybutyl, 4-Hydroxybutyl, 5-Hydroxypentyl, 6-Hydroxyhexyl, 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl, 2- oder 4-Butoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 4,8-Dioxadecyl, 3,6,9-Trioxadecyl, 3,6,9-Trioxaundecyl, 4,8,12-Trioxatridecyl, 2-Phenoxyethyl, 2-Phenoxypropyl, 3-Phenoxypropyl, 2-Phenoxybutyl, 4-Phenoxybutyl, 5-Phenoxypentyl, 6-Phenoxyhexyl, 5-Hydroxy-3-oxapentyl, 8-Hydroxy-4-oxaoctyl, 6-Phenoxy-4-oxahexyl, 2-Formyloxyethyl, 2-Acetyloxyethyl, 2-Propionyloxyethyl, 2- oder 3-Formyloxypropyl, 2- oder 3-Acetyloxypropyl, 2- oder 3-Propionyloxypropyl, 2- oder 4-Formyloxybutyl, 2- oder 4-Acetyloxybutyl, 2- oder 4-Propionyloxybutyl, 2-(Tetrahydropyran-4-yl)ethyl, 2- oder 3-(Tetrahydropyran-4-yl)propyl, 2- oder 4-(Tetrahydropyran-4-yl)butyl, Prop-2-en-1-yl, But-2-en-1-yl, 2-Methylprop-2-en-1-yl, Cyclopentyl, Cyclohexyl oder Cycloheptyl.

Reste X¹ und X² sind weiterhin z.B. Fluor, Chlor oder Brom.

Bevorzugt ist eine Verfahrensweise zur Herstellung von Diaminopyridinen der Formel I, in der X¹ Wasserstoff und X² Cyano bedeuten.

Weiterhin bevorzugt ist eine Verfahrensweise zur Herstellung von Diaminopyridinen der Formel I, in der R¹ Methyl bedeutet.

Weiterhin bevorzugt ist eine Verfahrensweise zur Herstellung von Diaminopyridinen der Formel I, in der R², R³ und R⁴ jeweils Wasserstoff bedeuten.

Weiterhin bevorzugt ist eine Verfahrensweise zur Herstellung von Diaminopyridinen der Formel I, in der R² Methyl oder Methoxy und R³ und R⁴ jeweils Wasserstoff oder Methyl, oder in der R² und R³ jeweils Methyl und R⁴ Wasserstoff bedeuten.

Weiterhin bevorzugt ist eine Verfahrensweise zur Herstellung von Diaminopyridinen der Formel I, in der R⁵ C₁-C₈-Alkyl, das durch 1 oder 2 Sauerstoffatome in Etherfunktion unterbrochen sein kann und gegebenenfalls durch Hydroxy oder Phenyl substituiert ist, oder Allyl bedeutet.

Im erfindungsgemäßen Verfahren wird der 1. Schritt in Gegenwart eines inerten organischen Verdünnungsmittels und/oder Wasser durchgeführt. Geeignete Verdünnungsmittel sind insbesondere solche, die mit Wasser entweder überhaupt nicht oder aber nur begrenzt mischbar sind. Beispielhaft seien Isobutanol, Toluol, o-, m- oder p-Xylol, Ethylbenzol oder deren Gemische genannt. Die Verwendung von Isobutanol, Toluol oder Xylol ist bevorzugt.

Geeignete Basen, die im 1. Schritt zur Anwendung kommen können, sind z.B. Alkalihydrogencarbonate oder -carbonate, wie Natrium- oder Kaliumhydrogencarbonat oder Natrium- oder Kaliumcarbonat. Die Verwendung von Natriumcarbonat ist bevorzugt.

Der 2. Schritt des neuen Verfahrens wird bei einem pH-Wert von 3,5 bis 6,5, vorzugsweise 4 bis 5,5, durchgeführt. Da die Bestimmung des pH-Werts in einer Schmelze schwierig ist, empfiehlt es sich, jeweils eine Probe der Schmelze in Wasser zu geben und den pH-Wert des wäßrigen Systems zu bestimmen. Die Einstellung des obengenannten pH-Werts kann sowohl mit organischen als auch mit anorganischen Säuren erfolgen. Geeignete Säuren sind z.B. Schwefelsäure, Salzsäure oder p-Toluolsulfonsäure. Die Verwendung von p-Toluolsulfonsäure ist bevorzugt.

Im erfindungsgemäßen Verfahren betragt das Molverhältnis Dichlorpyridin II : Amin III 1:1,03 bis 1:1,5, vorzugsweise 1:1,05 bis 1:1,25 und das Molverhältnis Dichlorpyridin II : Anilin IV 1:1,3 bis 1:3, vorzugsweise 1:1,5 bis 1:3.

Je mol Dichloroyridin II verwendet man im 1. Schritt üblicherweise 1 bis 2 Moläquivalent, vorzugsweise 1 bis 1,3, Moläquivalent einer Base.

Bezogen auf das Gewicht von Dichlorpyridin II kommen im 1. Schritt in der Regel 10 bis 150 Gew.-%, vorzugsweise 30 bis 100 Gew.-%, an inertem organischen Verdünnungsmittel zur Anwendung.

Zum Starten der Reaktion im 2. Schritt genügen in der Regel katalytische Mengen an Säure.

Das erfindungsgemäße Verfahren wird zweckmäßig so durchgeführt, daß man im 1. Schritt Dichlorpyridin II und inertes organisches Verdünnungsmittel vorlegt und unter Rühren zunächst auf eine Temperatur von 10 bis 80°C, vorzugsweise 45 bis 70°C und insbesondere 45 bis 60°C bringt. Zu diesem Gemisch wird dem bei der genannten Temperatur das Amin III und anschließend die Base gegeben. Danach wird bei der genannten Temperatur die Umsetzung durchgeführt. Wenn die Reaktion beendet ist, was im allgemeinen 12 bis 24 Stunden in Anspruch nimmt, kann das Reaktionsgemisch angesäuert, mit Wasser verdünnt und anschließend vom Verdünnungsmittel oder vom Gemisch Verdünnungsmittel/Wasser befreit werden. Dies kann beispielsweise durch Destillation geschehen. Das so zurückgewonnene Verdünnungsmittel kann in das Verfahren zurückgeführt werden.

Im 1. Schritt fallen die Diaminopyridine der Formel I als Isomerengemische an, die zu einem überwiegenden Teil aus einem Produkt der Formel I in der X¹ Wasserstoff und X² Cyano bedeuten, und zu einem geringen Teil aus einem Produkt der Formel I, in der X¹ Cyano und X² Wasserstoff bedeuten, bestehen, wenn man die Umsetzung ca. 12 Stunden bei einer Temperatur von bis zu 55°C durchführt.

Vorzugsweise wird das überwiegend anfallende Diaminopyridin I (X¹=H, X²=CN) vor der Abtrennung des Verdünnungsmittels und vor Beginn des 2. Schrittes praktisch isomerenfrei zwischenisoliert. Dies kann z.B. durch Abfiltrieren geschehen, da es beispielsweise bei Verwendung von Toluol als Verdünnungsmittel in der Regel als Niederschlag vorliegt.

Für die Herstellung solcher Diaminopyridine der Formel I, in der R⁵ einen Alkylrest bedeutet, der einen Hydroxygruppe aufweist, empfiehlt es sich in manchen Fällen, nicht von den entsprechenden Hydroxyalkylaminen III sondern von deren veresterten Verbindungen (C₁-C₄-Alkanoyloxyalkylverbindungen) auszugehen. Die Alkanoylgruppe kann anschließend wieder hydrolytisch abgespalten werden.

Durch Zugabe von Säure und Anilin IV zu der nach Abtrennung des Verdünnungsmittels resultierenden Schmelze erfolgt nun im 2. Schritt die Bildung der Diaminopyridine I. Der 2. Reaktionsschritt wird in der Regel bei einer Temperatur von 90 bis 165°C, vorzugsweise 125 bis 140°C, durchgeführt. Nach beendeter Umsetzung, die in der Regel 8 bis 20 Stunden in Anspruch nimmt, erfolgt die Aufarbeitung.

Diese kann auf an sich übliche Weise durchgeführt werden. Beispielsweise kann das Reaktionsgemisch mit Wasser und Isobutanol verdünnt und mittels einer Base, z.B. Natronlauge, neutralisiert werden. Danach wird Wasser, Isobutanol und überschüssiges Anilin IV unter Normaldruck abdestilliert. Restliches Anilin IV kann mittels Destillation unter vermindertem Druck aus dem Reaktionsgemisch entfernt werden. Es ist möglich das zurückgewonnene Anilin IV wieder in die Reaktion zurückzuführen.

Das resultierende Diaminopyridin der Formel I kann entweder in dieser Form weiterverwendet oder es kann noch einer Umfällung unterworfen werden. Dazu wird es in konzentrierter Salzsäure gelöst und anschließend mit Natronlauge wieder ausgefällt.

Das erfindungsgemäße Verfahren liefert die Diaminopyridine der Formel I auf einfache Weise und in hoher Reinheit, insbesondere hoher Isomerenreinheit, und Ausbeute.

Bei den Diaminopyridinen der Formel I handelt es sich um wertvolle Zwischenprodukte, insbesondere Kupplungskomponenten (X¹/X² = H, CN), für die Synthese von Farbstoffen.

Die folgenden Beispiele sollen die Erfindung näher erläutern.

### Beispiel 1

900 ml Isobutanol wurden bei Raumtemperatur mit einem Gemisch aus 935 g 2,6-Dichlor-3-cyano-4-methylpyridin und 180 ml Wasser verrührt. Dann erhöhte man die Temperatur auf 40°C und gab insgesamt 479 g 3-Methoxypropylamin unter leichter Kühlung innerhalb von 1 Stunde hinzu. Anschließend rührte man 2 Stunden bei 40 bis 45°C nach und versetzte dann mit 300 g Soda. Man rührte weitere 2 Stunden bei 40 bis 45°C und steigerte die Temperatur dann langsam auf maximal 80°C, bis die Umsetzung beendet war. Dann löste man die anorganischen Salze durch Zugabe von 1000 ml Wasser, säuerte mit Schwefelsäure auf einen pH-Wert von 1 an und trennte dann die untere wäßrige Phase, die den Überschuß des Amins enthielt, ab. Danach wurde die organische Phase auf einen pH-Wert von 4 bis 5 gestellt und anschließend Isobutanol destillativ entfernt. Bei 125°C gab man 1400 g o-Anisidin und 20 g p-Toluolsulfonsäure hinzu und rührte dann bei 130 bis 135°C. Während der Reaktion wurde der pH-Wert einer auf Wasser/Methanol ausgerührten Probe kontrolliert. Durch regelmäßige Zugabe von wasserfreiem Soda zum Reaktionsgemisch hielt man den pH-Wert des Reaktionsgemisches im Bereich von 4,5 bis 5,5. Insgesamt wurden 216 g Soda benötigt. Danach erhitzte man zur Beendigung der Reaktion für 6 Stunden auf 155°C. Anschließend kühlte man auf 110 bis 115°C ab, setzte langsam 60 g Wasser hinzu, wodurch der Reaktionskomplex Kohlendioxid freisetzte. Nach beendeter Kohlendioxid-Entwicklung stellte man den pH-Wert des Gemisches auf 7 bis 7,5 und destillierte den Überschuß an o-Anisidin bei vermindertem Druck nahezu quantitativ ab.

Der Rückstand wurde durch Versetzen mit wenig Ethylendiglykolmonomethylether kristallisiert und nach Ausfällen mit Wasser abfiltriert, gewaschen und getrocknet. Ausbeute: 1460 g eines Produktgemisches der Formel 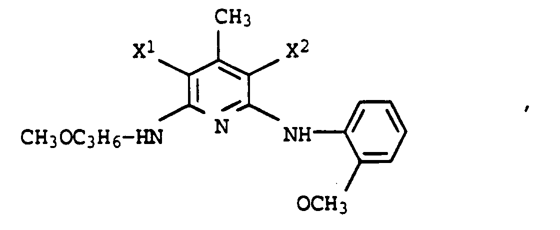 wobei das Isomerenverhältnis Produkt 1 (X¹ = H/X² = CN) : Produkt 2 (X¹ = CN/X² = H) 75 : 25 betrug.

### Beispiel 2

### 1. Schritt

374 g 2,6-Dichlor-3-cyano-4-methylpyridin wurden bei maximal 30°C in 1000 ml 70 gew.-%ige, wäßrige Ethylaminlösung eingetragen. Man rührte das Gemisch 3 Stunden bei 15 bis 30°C, fällte mit Eis, filtrierte den Niederschlag ab, wusch mit Wasser nach und trocknete. Man erhielt ein Gemisch von 2-Chlor-3-cyano-4-methyl-6-ethylaminopyridin (ca. 290 g) und 2-Ethylamino-3-cyano-4-methyl-6-chlorpyridin (ca. 96 g), das bei 90 bis 110°C schmilzt. Dieses Gemisch wurde in 600 ml Toluol heiß gelöst. Nach dem Abkühlen fiel ein grauweißes Produkt der Formel 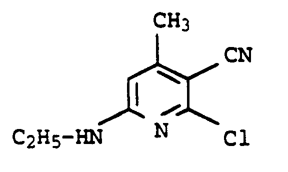 aus. Man isolierte es durch Absaugen. Ausbeute: 220 g Schmelzpunkt: 128°C

### 2. Schritt

Der 2. Schritt wurde analog Beispiel 1 durchgeführt. Als Anilinderivat verwendete man unsubstituiertes Anilin

Man erhielt 270 g eines Produkts der Formel 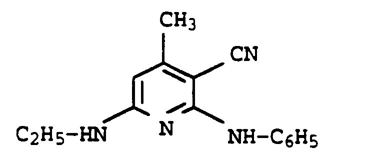
- Schmelzpunkt:: 96°C

### Beispiel 3

### 1. Schritt

440 g 2,6-Dichlor-3-cyano-4-methylpyridin wurden in einem Gemisch aus 1000 ml Toluol und 300 ml Wasser suspendiert. Dann gab man bei 36 bis 48°C unter Kühlung 230 g 3-Methoxypropylamin innerhalb von 1 Stunde hinzu und rührte das Gemisch 2 Stunden bei 45 bis 55°C nach. Dann gab man innerhalb von 20 min 141 g Soda hinzu und rührte 10 Stunden bei 45 bis 55°C und weitere 3 Stunden bei 75°C nach. Man säuerte anschließend mit konzentrierter Salzsäure auf einen pH-Wert von ca. 1,5 an und trennte dann die untere, wäßrige Phase ab. Dann kühlte man auf Raumtemperatur ab, filtrierte das ausgefallene 2-Chlor-3-cyano-4-methyl-6-(3-methoxypropylamino)pyridin ab und isolierte es analog Beispiel 2.

Man erhielt 370 g des isomerenreinen (3-Cyano)-Produktes mit einem Schmelzpunkt von 106°C.

### 2. Schritt

Der 2. Schritt wurde analog Beispiel 1 durchgeführt. Als Anilinderivat verwendete man o-Anisidin.

Man erhielt 507 g eines Produkts der Formel 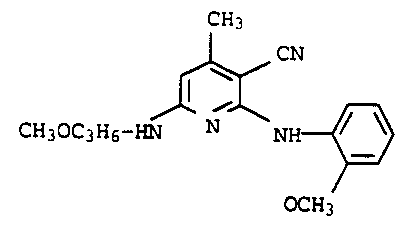
- Schmelzpunkt:: 128°C

In analoger Weise werden die in der folgenden Tabelle aufgeführten Verbindungen der Formel 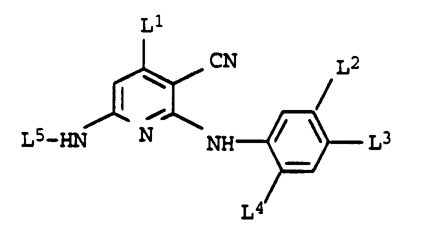 erhalten.

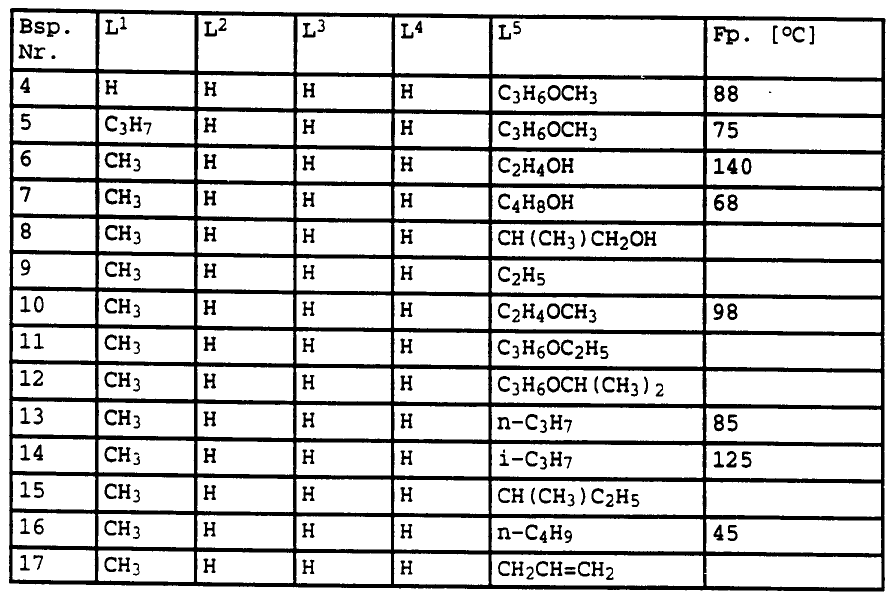

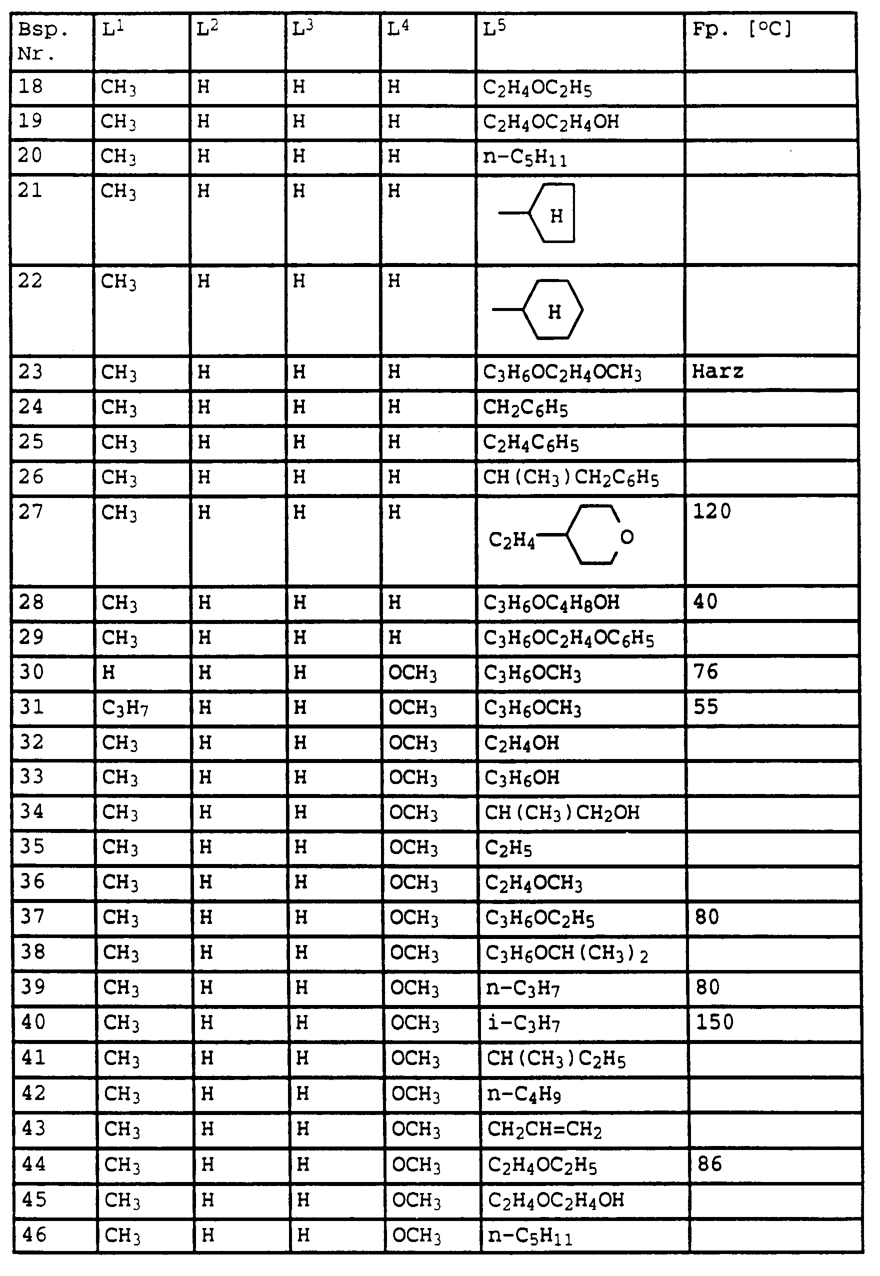

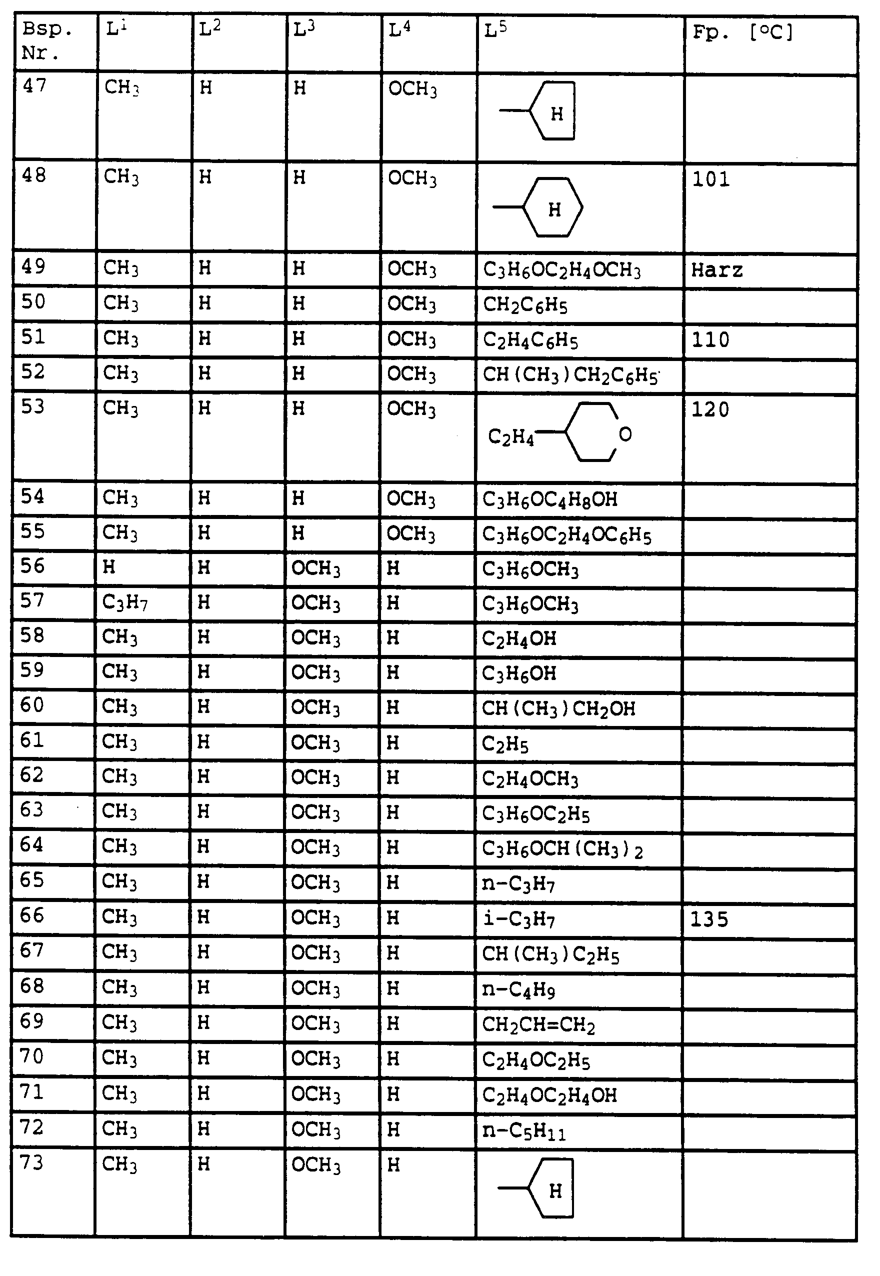

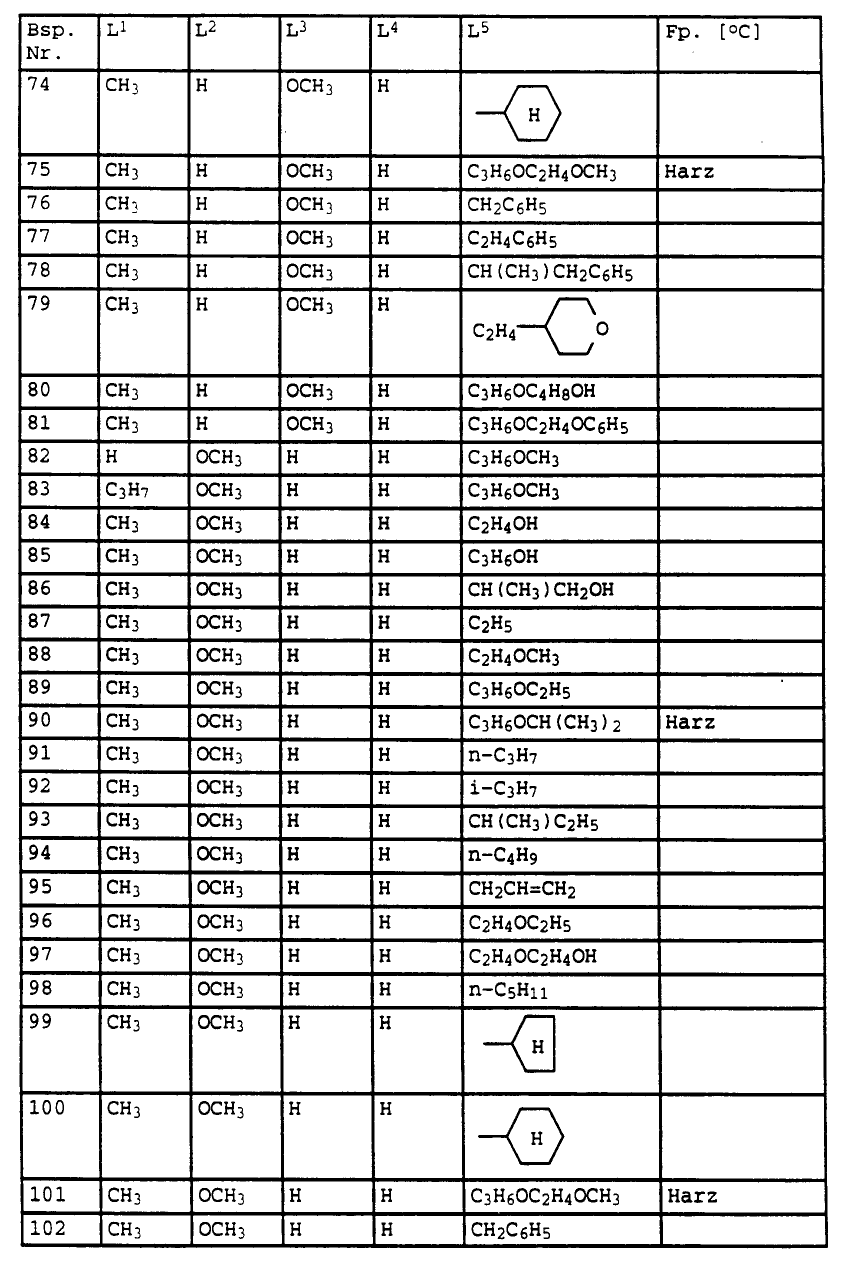

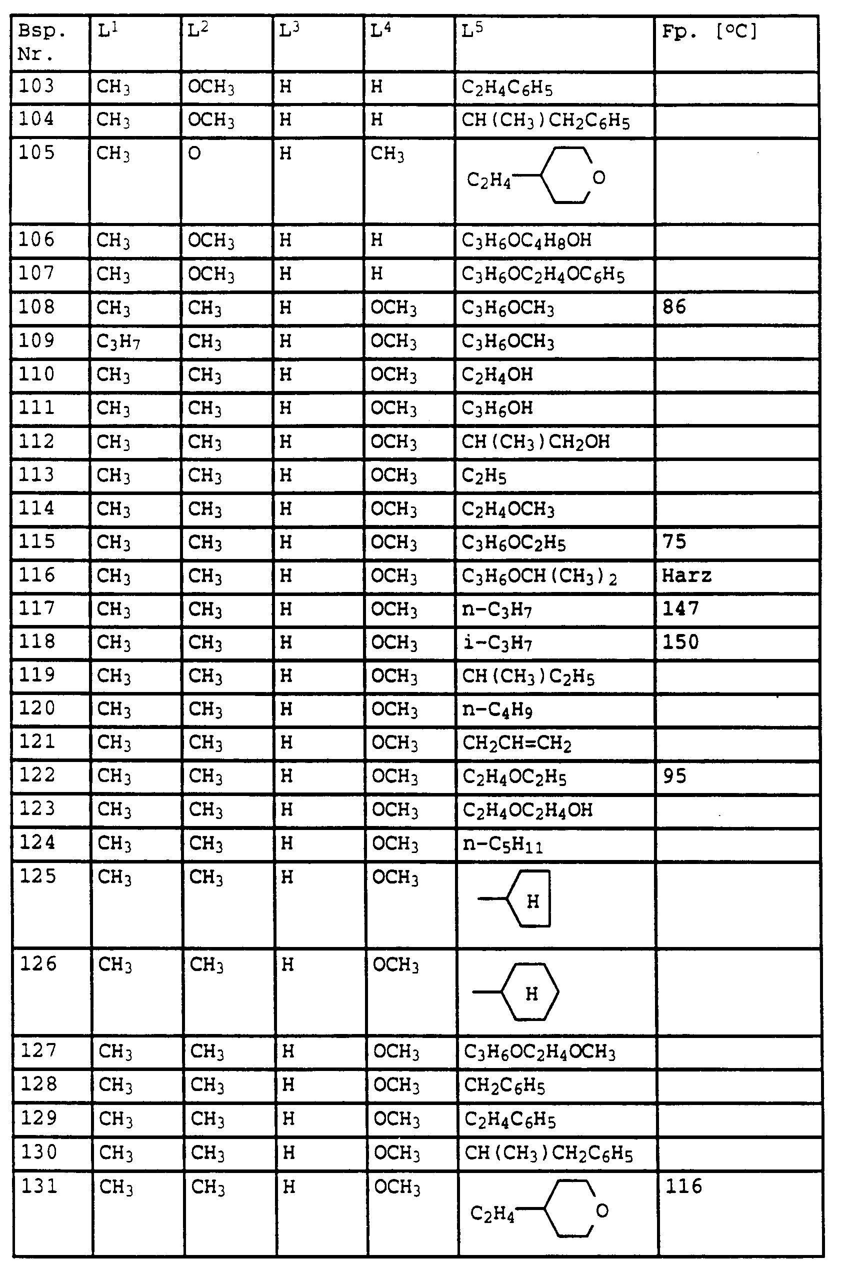

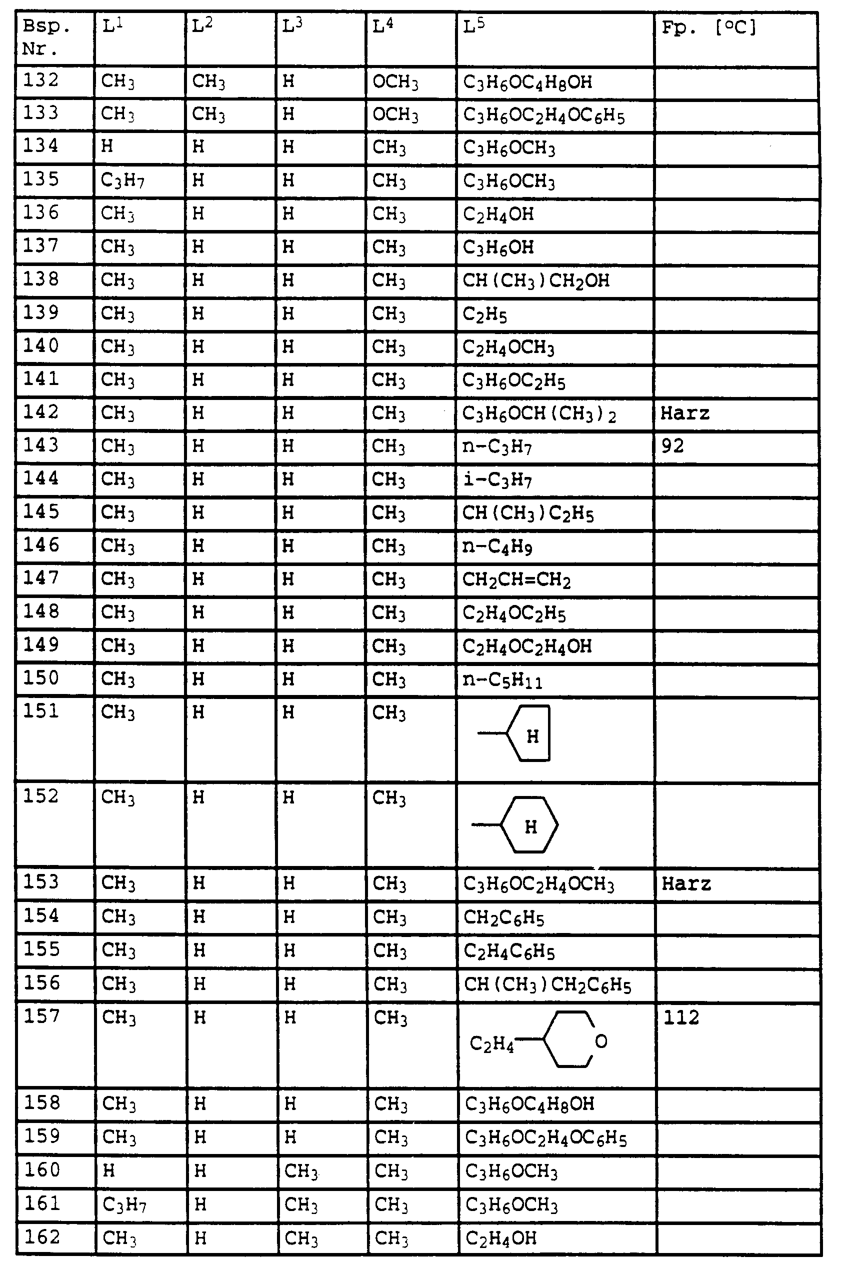

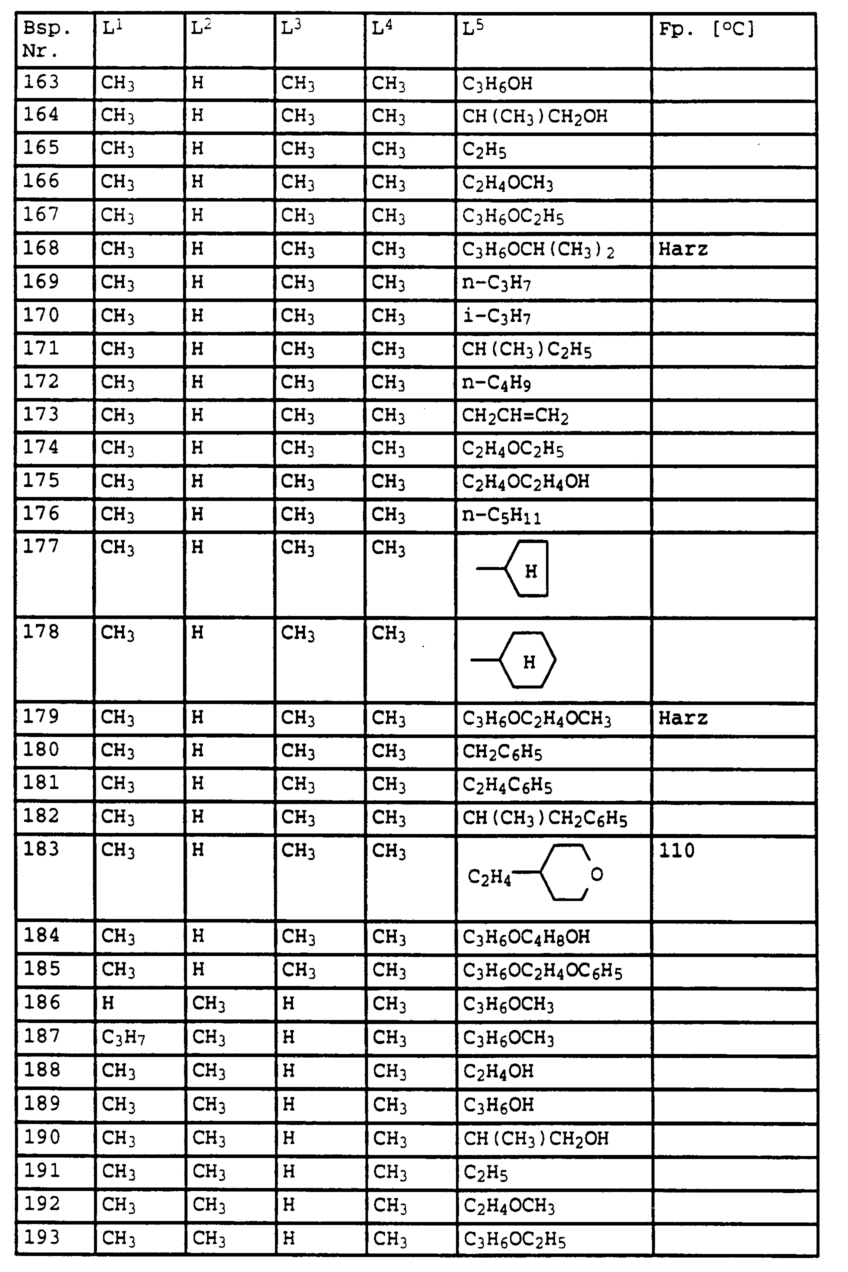

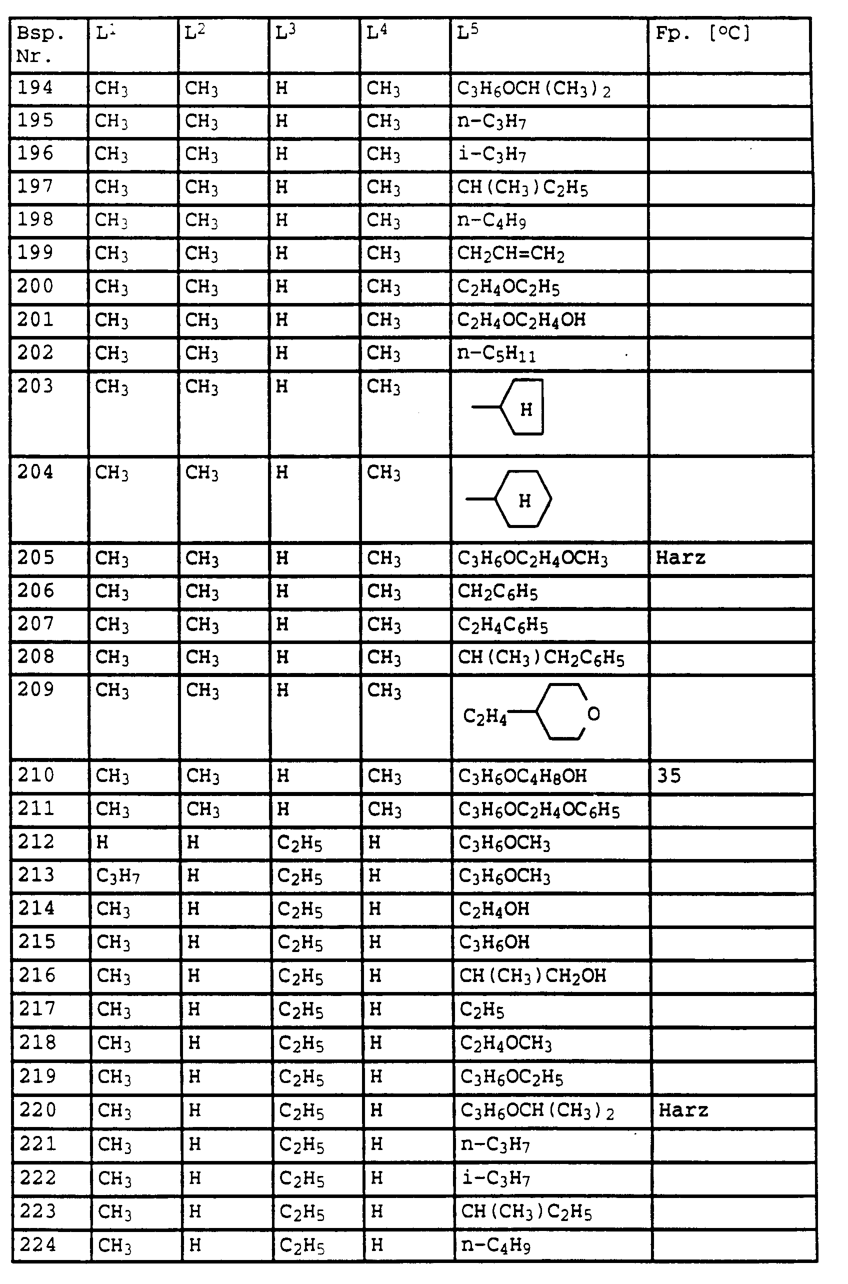

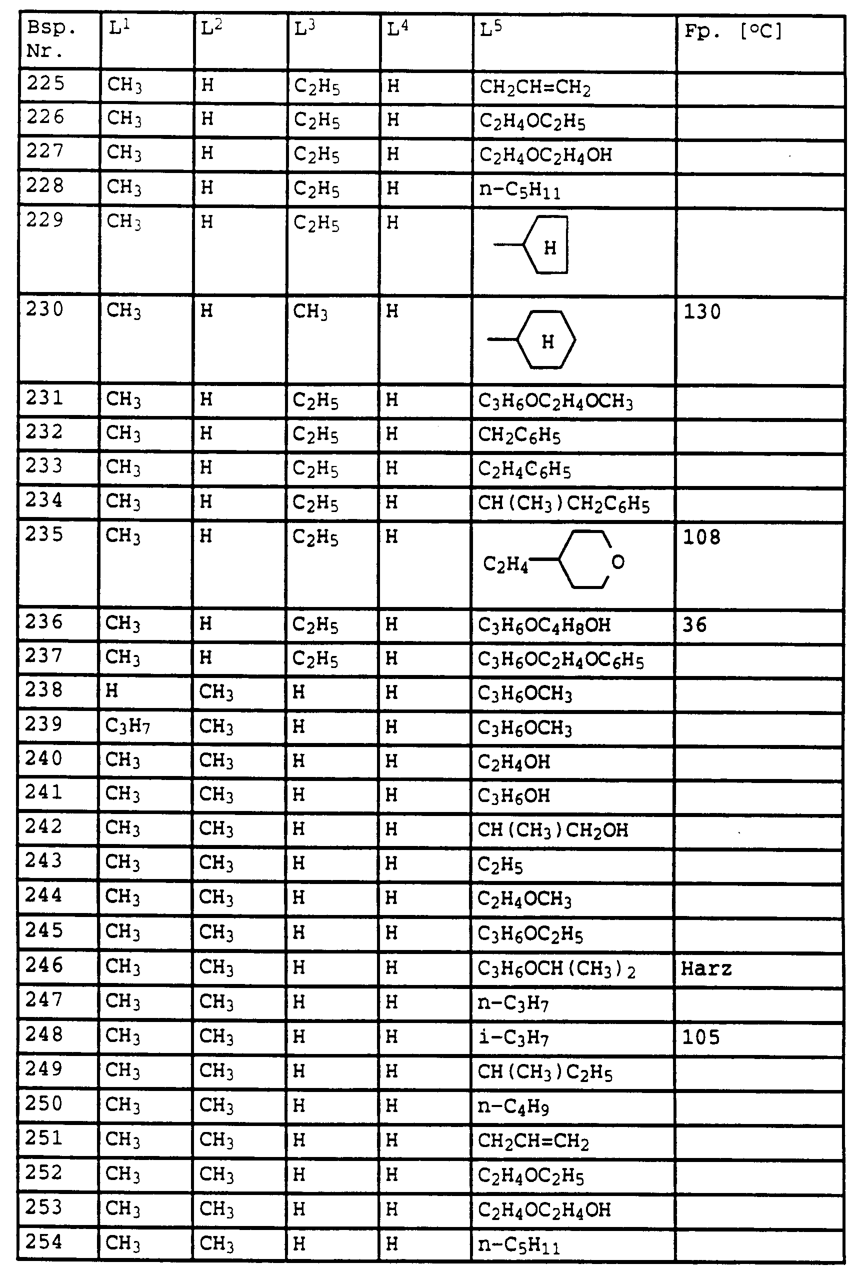

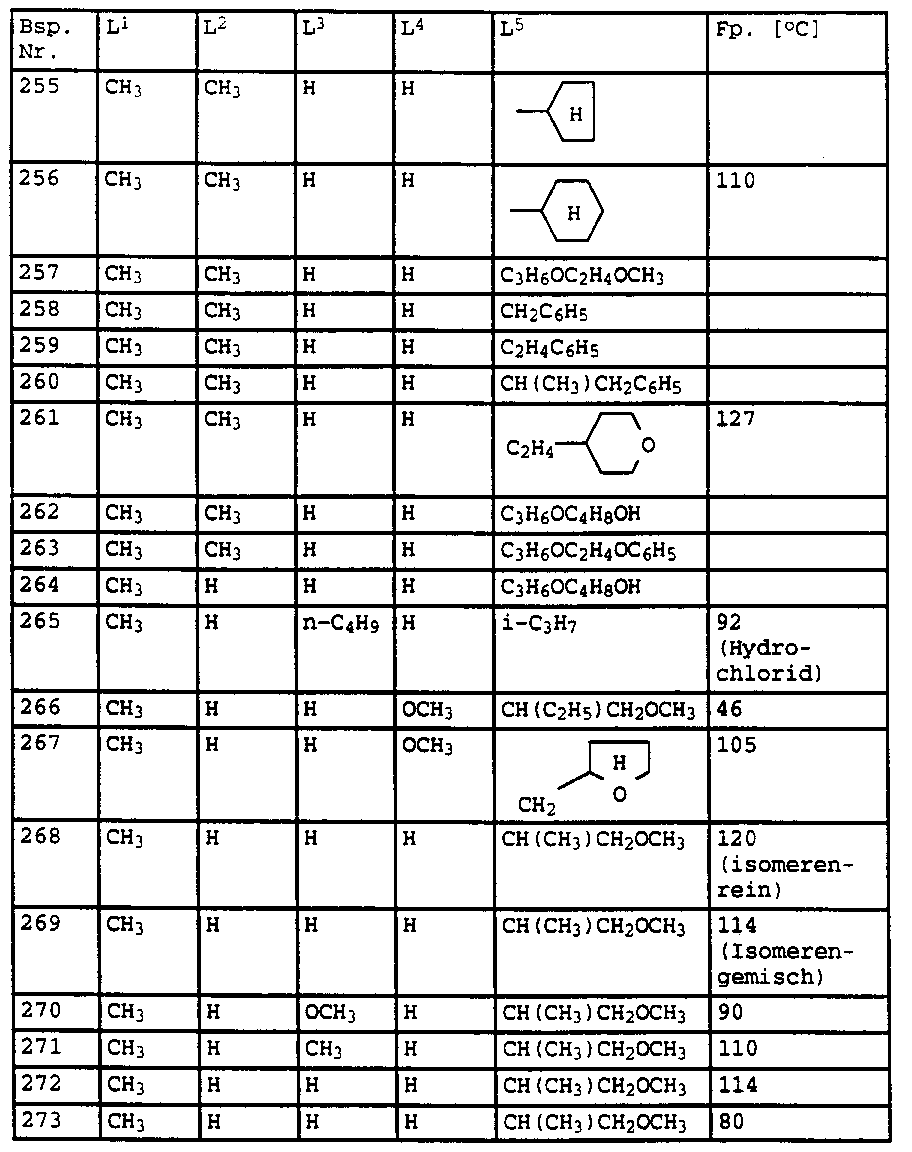

## Patentansprüche

1. Verfahren zur Herstellung von Diaminopyridinen der Formel I 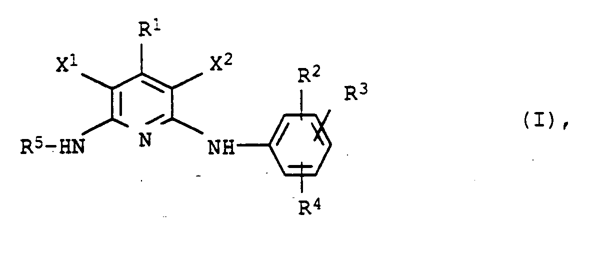 in der
einer der beiden Reste X¹ und X² Wasserstoff, C₁-C₄-Alkyl, Halogen oder Nitro und der andere Cyano,
R¹ Wasserstoff, C₁-C₄-Alkyl oder Phenyl,
R² und R³ unabhängig voneinander jeweils Wasserstoff, C₁-C₄-Alkyl oder C₁-C₄-Alkoxy,
R⁴ Wasserstoff oder C₁-C₄-Alkyl und
R⁵ C₁-C₁₀-Alkyl, das durch 1 bis 3 Sauerstoffatome in Etherfunktion unterbrochen sein kann und gegebenenfalls durch Hydroxy, C₁-C₄-Alkanoyloxy, Phenoxy, Phenyl, Tetrahydrofuranyl oder Tetrahydropyranyl substituiert ist, C₃-C₄-Alkenyl oder C₅-C₇-Cycloalkyl bedeuten,
durch Umsetzung von Dichlorpyridinen der Formel II 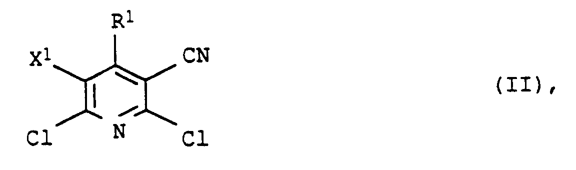 in der R¹ und X¹ jeweils die obengenannte Bedeutung besitzen, mit Aminen, wobei man in einem 1. Schritt ein Dichlorpyridin der Formel II mit einem Amin der Formel III
R⁵-NH₂ (III),
in der R⁵ die obengenannte Bedeutung besitzt, bei einer Temperatur von 10 bis 80°C in Gegenwart einer Base und eines inerten organischen Verdünnungsmittels und/oder Wasser umsetzt, anschließend vom Verdünnungsmittel befreit und danach, gegebenenfalls nach Zwischenisolierung des Reaktionsprodukts, in-einem 2. Schritt mit einem Anilin der Formel IV 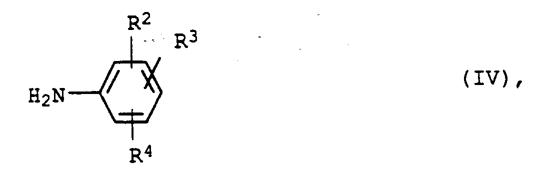 in der R², R³ und R⁴ jeweils die obengenannte Bedeutung besitzen, zur Reaktion bringt, dadurch gekennzeichnet, daß man den 2. Schritt in einer Schmelze bei einer Temperatur von 90 bis 165°C und bei einem pH-Wert von 3,5 bis 6,5 vornimmt, wobei je mol Dichlorpyridin II 1,3 bis 3 mol Anilin IV zur Anwendung kommen.

2. Verfahren nach Anspruch 1, dadurch gekennzeichnet, daß in Formel I X¹ Wasserstoff und X² Cyano bedeutet.

3. Verfahren nach Anspruch 1, dadurch gekennzeichnet, daß in Formel I R¹ Methyl bedeutet.

4. Verfahren nach Anspruch 1, dadurch gekennzeichnet, daß man im 1. Schritt Isobutanol, Toluol, o-, m-, oder p-Xylol, Ethylbenzol oder deren Gemische als Verdünnungsmittel verwendet.

## Claims

1. A process for preparing diaminopyridines of the formula I 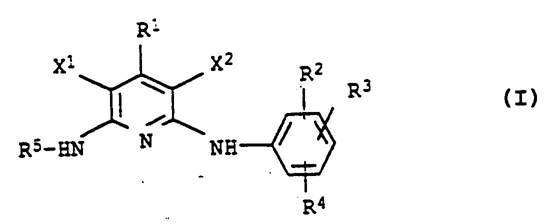 where
one of the two radicals X¹ and X² is hydrogen, C₁-C₄-alkyl, halogen or nitro and the other is cyano,
R¹ is hydrogen, C₁-C₄-alkyl or phenyl,
R² and R³ are each independently of one another hydrogen, C₁-C₄-alkyl or C₁-C₄-alkoxy,
R⁴ is hydrogen or C₁-C₄-alkyl, and
R⁵ is C₁-C₁₀-alkyl which may be interrupted by from 1 to 3 oxygen atoms in ether function and is optionally hydroxyl-, C₁-C₄-alkanoyloxy-, phenoxy-, phenyl-, tetrahydrofuranyl- or tetrahydropyranyl-substituted, C₃-C₄-alkenyl or C₅-C₇-cycloalkyl,
by reacting dichloropyridines of the formula II 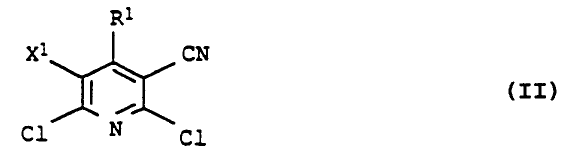 where R¹ and X¹ are each as defined above, with amines, by reacting in a first step a dichloropyridine of the formula II with an amine of the formula III
R⁵-NH₂ (III)
where R⁵ is as defined above, at from 10 to 80°C in the presence of a base and of an inert organic diluent and/or water, then removing the diluent and thereafter, with or without prior intermediate isolation of the reaction product, reacting it in a second step with an aniline of the formula IV 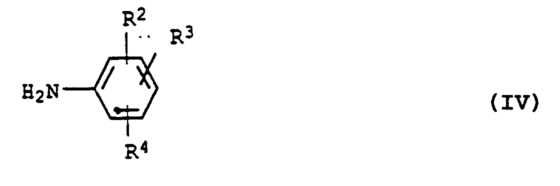 where R², R³ and R⁴ are each as defined above, characterized in that the second step is carried out in a melt at from 90 to 165°C and at a pH of from 3.5 to 6.5 using from 1.3 to 3 mol of aniline IV being used per mole of dichloropyridine II.

2. A process as claimed in claim 1, characterized in that in the formula I X¹ is hydrogen and X² is cyano.

3. A process as claimed in claim 1, characterized in that in the formula I R¹ is methyl.

4. A process as claimed in claim 1, characterized in that the diluent used in the first step is isobutanol, toluene, o-, m- or p-xylene, ethylbenzene or a mixture thereof.

## Revendications

1. Procédé de préparation de diaminopyridines de formule I 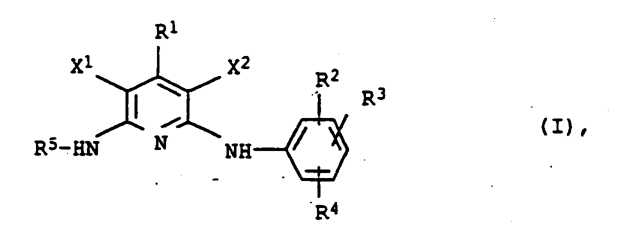 dans laquelle
l'un des deux restes X¹ et X² représente un atome d'hydrogène, un groupement alkyle en C₁-C₄, un atome d'halogène ou un groupement nitro, et l'autre représente un groupement cyano,
R¹ représente un atome d'hydrogène, un groupement alkyle en C₁-C₄ ou phényle,
R² et R³ représentent chacun, indépendamment l'un de l'autre, un atome d'hydrogène, un groupement alkyle en C₁-C₄ ou alcoxy en C₁-C₄,
R⁴ représente un atome d'hydrogène ou un groupement alkyle en C₁-C₄ et
R⁵ représente un groupement alkyle en C₁-C₁₀ pouvant être interrompu par 1 à 3 atomes d'oxygène en liaison éther et éventuellement substitué par des groupements hydroxy, alcanoyloxy en C₁-C₄, phénoxy, phényle, tétrahydrofuranyle ou tétrahydropyranyle, un groupement alcényle en C₃-C₄ ou cycloalkyle en C₅-C₇,
par réaction de dichloropyridines de formule II 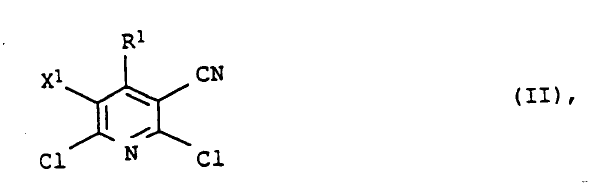 dans laquelle R¹ et X¹ prennent chacun la signification susmentionnée, avec des amines, et où on fait réagir, dans une première étape, une dichloropyridine de formule II avec une amine de formule III
R⁵-NH₂ (III),
dans laquelle R⁵ prend la signifcation susmentionnée, à une température de 10-80°C en présence d'une base et d'un diluant organique inerte et/ou d'eau, on élimine ensuite le diluant puis, après avoir éventuellement isolé le produit réactionnel intermédiaire, on l'amène à réagir, dans une deuxième étape, avec une aniline de formule IV 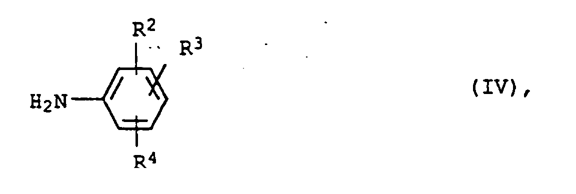 dans laquelle R², R³ et R⁴ prennent chacun la signification susmentionnée, caractérisé en ce que l'on effectue la deuxième étape dans une masse fondue à une température de 90-165°C et à une valeur de pH de 3,5 à 6,5, en utilisant 1,3 à 3 moles d'aniline IV par mole de dichloropyridine II.

2. Procédé selon la revendication 1, caractérisé en ce que X¹ représente un atome d'hydrogène et X² représente un groupement cyano dans la formule I.

3. Procédé selon la revendication 1, caractérisé en ce que R¹ représente un groupement méthyle dans la formule I.

4. Procédé selon la revendication 1, caractérisé en ce que l'on utilise, dans la première étape, de l'isobutanol, du toluène, de l'o-, m- ou p-xylène, de l'éthylbenzène ou leurs mélanges, en tant que diluant.
